# EUROPEAN PATENT APPLICATION

(11) **EP 4 521 417 A1**
(43) Date of publication of application: **12.03.2025**
(21) Application number: 23216304.8
(22) Date of filing: 13.12.2023
(51) Int. Cl.: G16H 50/20, A61B 5/08, A61B 5/00, G01N 33/497

(54) **METHODS AND SYSTEMS FOR TRAINING AI MODELS FOR DIAGNOSTICS**

(30) Priority: 08.09.2023 EP 23196387
(71) Applicant: Exhalation Technology Ltd, Cambridge, Cambridgeshire CB1 2JH (GB)
(72) Inventor: D'Cruz, Leon Gerard, Portsmouth, PO3 6LY (GB); BREJL, Stig Lytke, Cambridge, CB1 2JH (GB)
(74) Representative: ip21 Ltd

(57) **Abstract**

A method (S100) of obtaining training data for an existing artificial intelligence, AI, model to process data for diagnostics purposes, the method comprises the steps of: providing (S20) an input dataset comprising of a first set of data including data representative of a sampling device and a second set of data including time domain data representative of a patient sample; and processing (S30) the input dataset to obtain a transformed dataset to provide as training dataset to the existing AI model.

## Description

### Technical Field

This disclosure relates to using artificial intelligence models for diagnostics, and in particular for diagnostics of respiratory conditions. This disclosure further relates to the processing of datasets for training the artificial intelligence models.

### Background

Lung cancer is one of the deadliest cancers worldwide. Despite significant strides in systemic treatments for lung cancer like immunotherapy and advancements in stereotactic ablative radiotherapy (SABR), however survival rate continues to be strongly influenced by the stage at which diagnosis occurs. For instance, when diagnosed at stage 1, the estimated 1-year survival rate is 81.7%; however, if the diagnosis is made at stage 4, the average 1-year survival rate plummets to a mere 15.5%

The variability in lung cancer outcomes based on diagnosis stage underscores the critical importance of timely referrals for the correct treatment. Prompt intervention and treatment is required to decide whether to refer a patient for secondary or tertiary care within the 2-week wait rule. However, detecting lung cancer in its early stages is an arduous task primarily because its symptoms are diverse and non-specific, often resembling common respiratory issues associated with benign conditions.

Hydrogen peroxide (H₂O₂) is known as a crucial surrogate marker for identifying active inflammatory processes within the body, including pneumonia and lung cancer. H₂O₂ is generated in cells and tissues through enzymatic hydrolysis of the superoxide anion (O₂⁻), either within the mitochondrial electron transport chain or by NAD(P)H oxidases. H₂O₂ is detected in Exhaled Breath Condensate (EBC). EBC, obtained by cooling exhaled air, represents a liquid phase that closely mirrors the composition of the airway lining fluid (ALF).

It is known, particularly from the teachings of WO2022/248936**,** that it is possible to detect the presence and/or absence of certain components from EBC. The early detection of such components from individuals is an important part of assessing the health of that individual, as well as helping medical professionals to provide the correct treatment for identified conditions. The identification of components can have a huge benefit, particularly with conditions such as lung cancer, where the outcome of survival is strongly influenced at the stage of diagnosis.

At present, there remains a need for improved point of care (POC) applications for diagnostics.

### Summary

Aspects of the present invention are set out by the claims. Further aspects are also described.

In a first independent aspect, there is provided a method of obtaining training data for an existing artificial intelligence, Al, model to process data for diagnostics purposes, the method comprising the steps of: providing an input dataset comprising of a first set of data including data representative of a sampling device and a second set of data including time domain data representative of a patient sample; and processing the input dataset to obtain a transformed dataset to provide as training dataset to the existing Al model.

H₂O₂ acts as a biomarker for a variety of inflammatory conditions and oxidative stress in the airways. When combined with additional data, such as levels of CO₂ in exhaled breath, breath flow, breath temperature, breath relative humidity, it establishes a unique and data-rich "breath-print". This breath-print acquired from exhaled breath, processed using aspects according to aspects of the invention can provide a far more accurate diagnosis for individuals compared to prior solutions. Advantageously, the method allows for prompt analysis and interpretation of the data-rich "breath-print" acquired from the exhaled breath and correct identification of potential health conditions. In turn, this allows for point of care (POC) applications.

In particular, the method combines a first set of data including data representative of a sampling device, which can include several respiratory markers being logged by the device during sample collection, with a second set of data including time domain data representative of a patient (e.g. H₂O₂ from an EBC sample collection), to analyse whether patterns across these can provide higher levels of specificity as well as sensitivity. This requires processing and analysis of many large datasets including a variety of factors to offer a suitable diagnosis. These datasets would take a human either too long to process, or would be physically impossible for someone to establish trends in the data which could indicate certain health issues, such as lung cancer or pneumonia. The invention enables heavy processing power in a timely manner, the response time making it suitable for POC applications. The training and validation phases of the model may be advantageously completed in advance and saved.

Whilst the obtained diagnostics tool can be applied particularly to respiratory conditions, it will be appreciated that it can be applied to other conditions as well. Exhaled breath is a rich source of biomarkers and pathogens, providing insights in the health status of a test subject. Renal diseases are known to result in biomarkers being present in the exhaled breath. Different proteins being present in exhaled breath make give evidence of different cancer forms, not just lung cancer. Many infectious diseases spread through exhaled breath e.g., influenza and SARS-CoV-2. Further, cardiologic diseases may also result in respiratory symptoms and may be potentially diagnosed in a similar manner. Accordingly, the methods and systems according to the invention have applications for diagnostics not necessarily limited to respiratory conditions.

Preferably, the first dataset comprises of single point data values, for example, maximum CO₂ concentration and/or H₂O₂ concentration and/or maximum temperature from the EBC. It would be appreciated that single point data can include single time-point data, derived from and representing time tagged series, whilst other single point data are time independent. Alternatively, or in combination with, the single point data values may include: metadata related to the individual and/or additional samples, for example, sputum and/or blood and/or pleural fluid and/or saliva and/or nasal swab and/or throat swab samples.

Preferably, the Al model is a statistical artificial intelligence model. Even more preferably, the artificial intelligence model is a Bayesian network, for example, a Latent Dirichlet Allocation (LDA) model.

In other words, an initial model may be based on LDA. LDA, though not strictly Bayesian, incorporates Bayesian principles in its probabilistic approach to classification and dimensionality reduction. LDA models class data distributions with normality assumptions and equal covariances as well as estimating parameters using probability theory. It employs Bayesian decision theory, calculating posterior class probabilities based on observed data and prior information. This makes LDA statistically robust, accommodating noisy data and offering interpretability through optimal projections that reveal feature relevance. LDA's linear projections can aid in data visualization, aiding exploratory analysis and model selection. In essence, LDA's connection to Bayesian principles enhances its ability to handle uncertainty, provide insights, and create optimal decision boundaries in classification tasks.

In a dependent aspect, the Al model employs advanced data augmentation techniques. Preferably, the data augmentation techniques are used to address class imbalances from training and validation datasets.

Preferably, this would involve Synthetic Minority Over-Sampling Technique (SMOTE). Advantageously, this technique allows for the generation of synthetic samples for minority classes. Preferably data augmentation techniques, for example SMOTE, are applied to both the training and validation datasets. Advantageously, this further reduces any class imbalance seen within the model as both the training and validation datasets and improves the model's prediction.

In a further dependent aspect, the LDA model comprises at least one hyperparameter tuning technique. Preferably, the hyperparameter tuning technique is a grid search technique. Advantageously, the hyperparameter tuning technique improves the classification accuracy of the LDA mode.

In a dependent aspect, the LDA model is used to classify individuals into a distinct medical category. Preferably, the medical categories are related to respiratory conditions, for example, lung cancer and pneumonia.

In a dependent aspect, the LDA model is capable of generating custom reporting and visualisation outputs. Preferably, the reporting and visualisation outputs are related to the model's performance. Advantageously, this provides healthcare professionals a visual aid to ensure the LDA model is performing well. Alternatively, the output may relate to the diagnostic output. Advantageously, this provides a healthcare professional with visual representation to interpret the diagnostic results generated by the LDA model.

In a further dependent aspect, the LDA model output is capable of being stored on a cloud-based database. For example, such cloud-based databases are platforms such as, but not excluded to, Amazon Web Service (AWS) and/or Microsoft Azure, and/or Google Cloud Platform (GCP).

In a further dependent aspect, the LDA model is capable of being implemented on a mobile application. Preferably, the mobile application is compatible with iOS and Android operating systems.

In a dependent aspect the input data comprises of single point data values and time domain data. Examples of time domain data include, breath temperature and/or breath flow rate and/or breath humidity and/or CO₂ concentration over a specified time period.

It is known that single point data values are a challenge for certain AI models, such as Convolutional Neural Network (CNN) models, to process. Single point data is not typically used directly with CNN models as they are primarily designed for processing grid-like data, such as images, where the network learns hierarchical features from local receptive fields. Single point data is often better suited for other types of neural networks or machine learning models like: feed forward Neural Networks; Decision Trees and Random Forests; Gradient Boosting models; Recurrent Neural Networks; Transformer-based models which are used for natural language processing tasks; or AutoML-Tools.

Linear algebra involves mathematical operations with matrices and vectors. When adapting tabular data for CNNs, linear algebra is used to reshape the data into a matrix format, where each row corresponds to an image. It helps transform tabular data into a suitable input format for image-based CNN algorithms in patent analysis, facilitating complex pattern recognition.

The conversion of tabular data to data matrices using linear algebra is one method option of feeding the data into a CNN network. However, converting data into an image format, such as using fractals, can have advantages in certain situations compared to converting tabular data into matrices and feeding them into CNN networks:
- Pattern Detection: Fractal-based representations can be particularly useful when data exhibits complex spatial or hierarchical patterns that are not easily captured by traditional tabular or matrix representations. Fractal patterns can encode rich and self-replicating structures, which CNNs might detect effectively.

Non-Euclidean Data: For data with inherent non-Euclidean properties, such as networkgraphs or irregularly shaped data (Clinical biomarker data for patients with irregularly scheduled measurements is an example of irregularly shaped data. For example, EBC readings taken at different and unstructured time points for each patient), fractal representations may provide a way to transform them into a regular image-like format that can be processed by CNNs.

Preferably, the artificial intelligence model trained is a deep learning artificial intelligence model. Preferably, the deep learning artificial intelligence model is a feed-forward neural network, for example a CNN model. CNN models are known and have the ability to handle diverse data types, including transformed data representations, whereby they can effectively interpret and process images. CNN models are able to identify intricate patterns and relationships within a dataset comprising of images, which enhances the ability to accurately classify individual input data into a specified category, such as a specific respiratory condition. Preferably, the cross-validation of the accuracy of the CNN model is greater than 80%.

A known method for working with time domain data and artificial intelligence models is to use the average values from the time domain data. However there are many disadvantages to using this method, including:
- loss of information by simplifying data;
- sensitivity to outliers which disproportionately represent the average;
- biased data as averages do not represent the underlying data distribution;
- diminished model robustness when faced with noisy data;
- limited feature engineering to improve model performance;
- ineffective handling of categorical data;
- averages may not capture the context in which data was collected;
- averages can lead to an overgeneralisation of the model;
- averages can obscure temporal patterns and dependencies;

Therefore, alternative methods of converting time-domain data to a suitable input data format is required to ensure a robust and highly predictive Al model.

In a dependent aspect, the step of processing the input dataset comprises: generating frequency domain data from time domain data in the second dataset; generating single point image data from the first dataset; and combining the frequency domain dataset with the generated single point image data.

Preferably, the method step generating the frequency domain data from time domain data comprises of a Fourier Transformation method. Preferably, Fast Fourier Transform (FFT) is used as this allows for faster and more efficient computation of transforming the data. Even more preferably, a 1-sided FFT method is performed. Breath-prints vary between individuals, for example the start and stop times of exhalation. Advantageously, the transformation of data from time domain to frequency domain allows harmonisation between individual's input data for the training and validation of the Al model. The FFT step can measure phase data and phase shifts which aids feature extraction for the Al model. With respect to respiratory data, transformation of the phase data extracted via FFT allows for identification of novel breathing patterns between individuals and is of particular use in medical devices, diagnostics and respiratory health monitoring technologies as it can aid in the identification of novel breathing patterns. Advantageously, the transformation of the data to a frequency domain also improves the trained artificial intelligence model's predictions as frequency domains are known to improve the reading of data which is not uniformly sampled and when timing of events does not affect the resulting output. Advantageously, Fourier transforms break down signals into their constituent frequencies, making it easy to analyse periodic behaviours which are common in medical results, such as capnogram, flow and pressure analysis. Advantageously, representing waveforms as Fourier transforms helps to distinguish signal from noise within the data which is essential for clean data interpretation and ensures that the data used for analysis or decision-making is accurate and reliable. Advantageously, FFT makes computing Fourier transforms of large datasets computationally efficient for real-time applications, such as a phone application. Advantageously in Machine Learning methods, Fourier transforms can be used to extract features for classification and regression. Advantageously, Fourier transforms reverse the effects of convolution operations, removing distortion in image processing and optics. Advantageously, Fourier transforms can simplify the overall Al model by working with frequency domain data. Advantageously, Fourier transforms allow for cross-correlation and convolution operations to be carried out in deriving metrics for machine learning processes.

In a dependent aspect, the Al model employs advanced data augmentation techniques. Such techniques could include, but are not exclusive to, rotation, shifting, shearing, zooming and flipping of images. Advantageously, this increases the diversity of the training dataset and improves overall performance of the model. In a further dependent aspect, data augmentation techniques are used to address class imbalances from training and validation datasets. Preferably, this would involve Synthetic Minority Over-Sampling Technique (SMOTE). Advantageously, this technique allows for the generation of synthetic samples for minority classes. In a further dependent aspect, advanced data augmentation techniques are applied to both the training and validation datasets. Advantageously, this further reduces any class imbalance seen within the model as both the training and validation datasets and improves the accuracy of the model's prediction.

In a further dependent aspect, the architecture of the CNN model comprises of convolutional layers with increasing complexity. Preferably, such layers include max-polling layers, global max-pooling and fully connected layers. Advantageously, these hyperparameters result in an increased performance from the CNN model when classifying images associated with medical conditions.

In a dependent aspect, the Al model comprises of a validation monitoring strategy module, wherein the validation monitoring strategy module is able to monitor validation accuracy during training and save the best-performing model. Furthermore, this ensures that the trained model is optimised for generalisation. In a further dependent aspect, the validation monitoring strategy comprises of a custom callback module, wherein the custom callback modules is able to recall the best model based on validation accuracy.

In a dependent aspect, the Al model comprises of an optimisation algorithm. Preferably, the optimisation algorithm is a Stochastic Gradient Descent (SGD). Even more preferably, the SGD comprises of a specific learning rate for optimisation. Advantageously, these can significantly improve the prediction accuracy of the model.

In a dependent aspect, the AI model is capable of generating custom reporting and visualisation outputs. Preferably, the reporting and visualisation outputs relate to the performance of the model. Advantageously, this provides healthcare professionals a visual aid in order to ensure the model is performing well. Alternatively, the output may relate to the diagnostic output. Advantageously, this provides a healthcare professional with visual representation to interpret the diagnostic results generated by the Al model.

In a dependent aspect, the Al model is used to diagnose individuals. Preferably, the medical categories are related to respiratory conditions, for example, lung cancer and pneumonia.

Preferably, the input data comprises components collected from breath condensate. Preferably the breath condensate is exhaled breath condensate (EBC).

In a dependent aspect the sampling device is adapted to combine measurements made on exhaled breath in its gas phase with measurement of H2O2 levels made on exhaled breath condensate in its liquid phase. In this case, the collection (S20) of the input data may be carried out by a device such as that disclosed in WO2022/248936 or Inflammacheck^{®}. The complex and rich initial data from such a device, detects components within an individual's breath-print.

When testing in exhaled breath, different targets take an outset in the analysis of different aspects of the exhaled breath, including, for example:
- Physical aspects - e.g., flow, temperature, relative humidity, patterns etc.
- Gas phase compounds - e.g., CO2, fractionated Nitric Oxide FeNO etc.
- Exhaled Breath Condensate - e.g., H2O2, pH value etc.
- Aerosols - e.g., different proteins, pathogens like SARS-CoV-2 etc.

When testing for H2O2 with the Inflammacheck^{®} the three first aspects listed above are targeted. However, it is also possible to use the or Inflammacheck^{®} for collection of aerosols by replacing a filter on the inlet by a saliva trap.

In a further dependent aspect, the Al model output is capable of being stored on a cloud-based database. For example, such cloud-based databases are platforms such as, but not excluded to, Amazon Web Service (AWS) and/or Microsoft Azure, and/or Google Cloud Platform (GCP). In a further dependent aspect, the Al model is capable of being implemented on a mobile application. Preferably, the mobile application is compatible with iOS or Android operating systems.

In an alternative dependent aspect, the LDA model and CNN model are combined. Preferably a fused model is generated. Advantageously, this will increase the prediction power for identification of diseased individuals and for diagnosis.

In a further independent aspect there is provided a system comprising a processor configured to perform the steps of: providing an input dataset comprising of a first set of data including data representative of a sampling device and a second set of data including time domain data representative of a patient sample; and processing the input dataset to obtain a transformed dataset suitable for providing as training data to an existing artificial intelligence, Al, model.

In a further independent aspect there is provided a diagnostic system comprising a system as described above.

In a further independent aspect there is provided a diagnostics method comprising a method according to claim 1.

### Brief Description of the Drawings

Embodiments will now be described, by way of example only, with reference to the accompanying drawings, in which:
- FIG. 1 illustrates a scatter-plot matrix of variables collected from an Inflammacheck^{®} device.
- FIG. 2A-C is a schematic illustrating a system configured to train an artificial intelligence model to analyse and interpret a dataset from collected EBC with further examples of graphical information.
- FIG. 3 is a flowchart that identifies a way of training of an Al model for diagnostic predictions.
- FIG. 4 is a flowchart that identifies a way of performing training of an Al model for diagnostic predictions with time domain and single point data values.
- FIG. 5A and 5B are a generated QR code and a fractal image respectively of a single point data value.
- FIG. 6 is a flowchart that identifies a method for generating the frequency domain data.
- FIG. 7 is a flowchart identifies a way of training a deep learning Al model for diagnostic predictions.
- FIG. 8A and 8B are a model accuracy graph and model loss graph respectively from a CNN model.
- FIG. 9 illustrates a method of building and training a statistical LDA machine learning model.
- FIG. 10A-G illustrates the diagnostic prediction effectiveness of two different LDA machine learning models.

### Detailed Description

Various exemplary embodiments, features, and aspects are described in detail below with reference to the drawings.

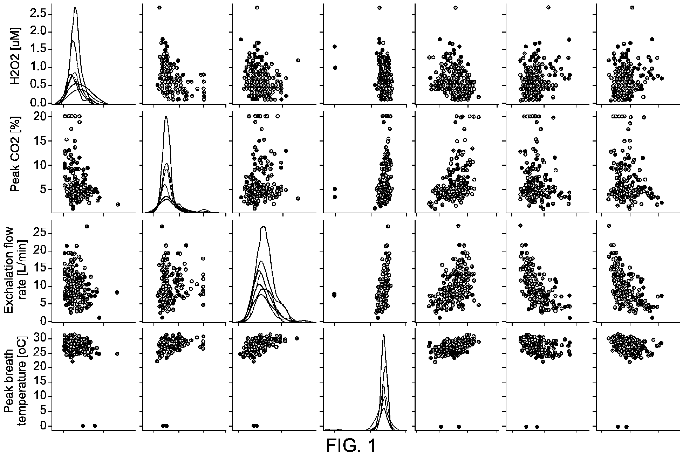

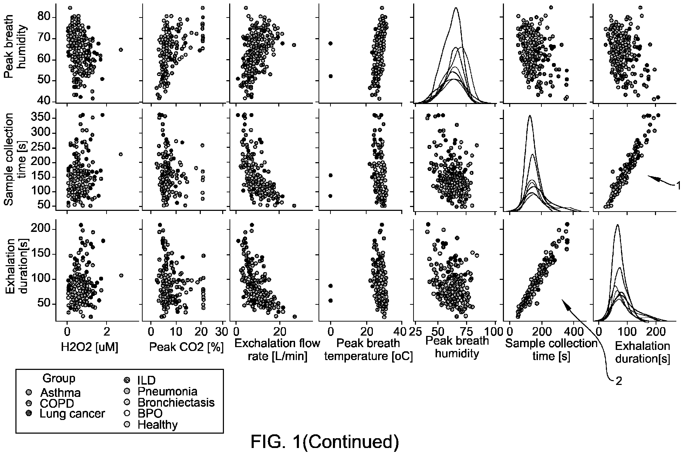

FIG. 1 shows a scatter-plot matrix of variables collected from an Inflammacheck^{®} device. Initial analysis involved the comparison of standardised Inflammacheck^{®} variables against one another, revealing bivariate relationships among various combinations of these variables, all derived from the Inflammacheck^{®} device's output. The comparison between exhalation duration 1 and sample collection time 2 variables exhibit a pronounced and positive correlation.

Unlike exhalation duration and sample collection time, the remaining variables were distributed in a scattered manner across the plots. This random distribution shows no direct linear relationship or strong correlation among the majority of the variables tested. Additionally, the distribution curves for such variables predominantly displayed a right-skewed pattern. Right-skewed distributions typically indicate that the mean values are greater than the medians. No clear clustering or demarcation between different disease classes based on the value ranges of the Inflammacheck^{®} variables was clearly identified. This suggests that these variables alone would not be sufficient to distinctly separate various pathological conditions from each other within the dataset and an artificial intelligence model may be able to distinguish and correlate.

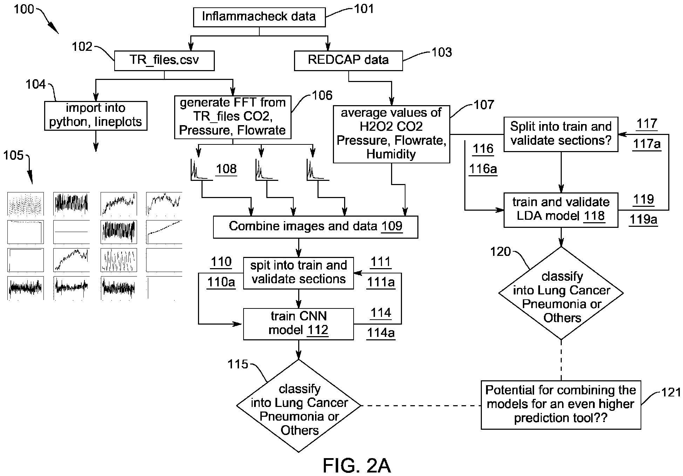

FIG. 2A illustrates a system 100 configured to process a breath-print dataset from exhaled breath condensate (EBC). The EBC is collected via a device, not shown, from exhalation of a patient over a continuous two-minute testing period whereby the device is capable of monitoring components found in EBC. Examples of such devices include an Inflammacheck^{®} device or one disclosed in WO2022/248936 incorporated herein by reference.

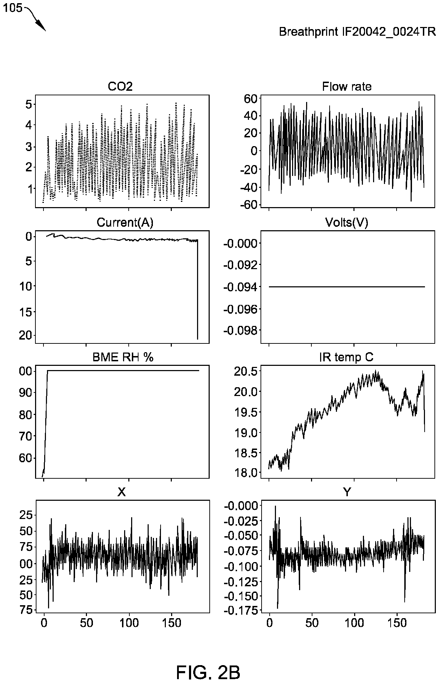

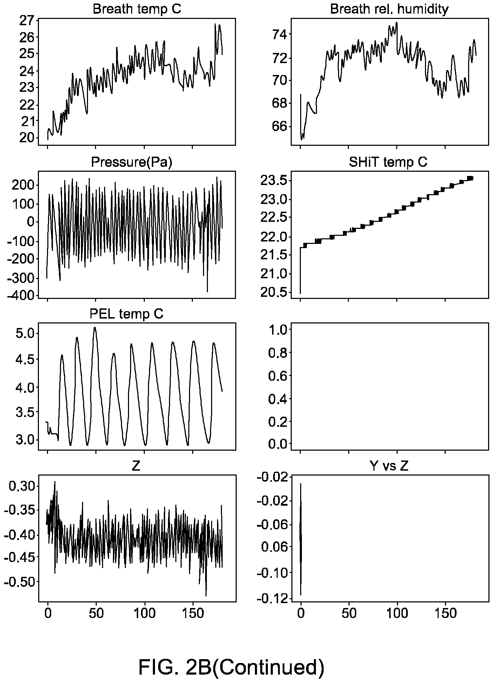

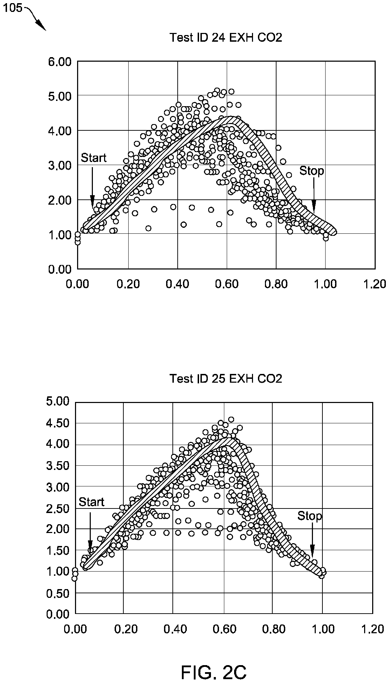

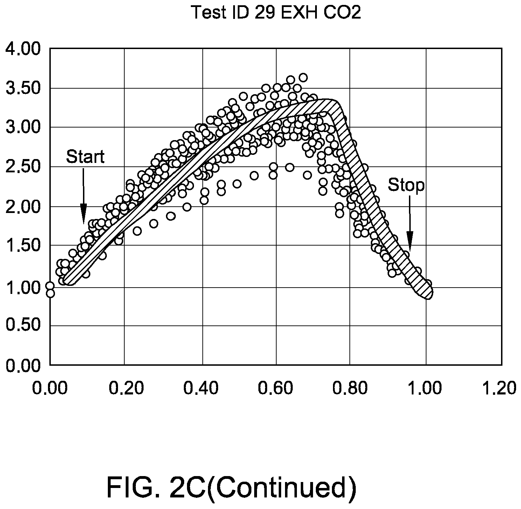

The input, initial dataset 101 is fed into the system. The initial dataset 101 comprises two subsets of data, including time domain data 102 and tabular data 103. The time domain data 102 can be imported into an Integrated Development Environment (IDE) 104 and can be analysed using well-known programming modules 104, such as Python modules. The time domain data 102 undergoes analysis and can be presented in a graphical format 105, such as value against time which can be seen in more detail in FIG. 2B. FIG. 2C depicts an alternative graphical format 105, of a time domain value 102 for three individuals. The graphs show the different start and stop points of exhalation for each individual is different. Advantageously, this can be analysed by professionals if so desired to visually look for abnormal patterns in an individual's data or it can be used by engineers to ensure the EBC monitoring device is functioning normally.

The time domain data 102 can also be transformed for further processing and analysis. The time domain data 102 undergoes Fast Fourier Transformation (FFT) 106 where the time domain data 102 is transformed from a time domain to a frequency domain. The FFT takes the time domain data 102 (i.e. CO₂ concentration over a two-minute period) and converts it into a frequency domain representation, revealing the underlying frequency components that make up the signal. There are many advantages to representing the time domain data 102 from a time domain signal to a frequency domain signal via FFT. Using FFT data enhances signal analysis, feature extraction, and problem-solving across diverse domains. The FFT data is then further presented in graphical format, such as a frequency domain graph 108.

The tabular data 103 comprises of a log file containing single point data values 107 for each test performed on a specific device on a specific date. The H₂O₂ concentration of the EBC, the average flow logged during exhalation, the maximum CO₂ levels / maximum breath temperature / maximum breath relative humidity logged over a two minute testing period are examples of single point data values 107. The tabular data 103 contains other test specific loggings which is capable of characterising each individual test.

More specifically, in an example, a test performed with Inflammacheck^{®} comprises different stages:
- Sample collection stage:
   ∘ During this stage a test subjects breathes through the Inflammacheck^{®} and EBC builds up in a cooled condensation area.
   ∘ The duration of this stage depends on how much time it takes for the individual to generate enough EBC sample in the condensation area for the test cycle to move into the next stage (HzOz measurement).
   ∘ Typically, it takes 1-3 minutes.
   ∘ During the sample collection stage, Inflammacheck^{®} measures and logs CO₂ content, flow, temperature, and relative humidity of the exhaled breath every 10 seconds.
   ∘ These time tagged loggings are forming the rich breath print jointly with the H₂O₂ reading.
- H₂O₂ measurement stage
   ∘ When enough EBC has been collected, the test subject is notified and prompted to place the device on a table.
   ∘ The device then performs a test assay targeting H₂O₂ contents which results in a single H₂O₂ level being determined and logged.

The single point data values 107 and frequency domain graphs 108 undergo+ further processing whereby the graphical representation depicting the frequency domain data 108 is combined with the corresponding single point data values 107 to generate combined data 109. In order for the combined data 109 to comply with the further machine learning models, the single point data values 107 must be converted from tabular data into a single image. A preferred example would include the use QR coding which allows for the conversion of single point data values 107 into a single QR code image. An alternative example, could comprise of encoding the single point data values 107 into fractal images. Preferably, a method for data cleansing and selection will be deployed if fractal images are used. The combined data 109 is then split into a training dataset 110 and a validation dataset 111.

The training dataset 110 is used to train an existing Al model, preferably a deep-learning model capable of image and video related tasks, such as a Convolutional Neural Networks (CNN) 112. It will be appreciated that several suitable models may be used.

Known CNN- neural networks typically include the following components:
**Convolutional Layers:** Convolutional layers are used to scan data provided to the CNN(e.g. an image) with small filters or kernels. These filters may detect specific features like edges, textures, or patterns by sliding across the input.
**Pooling Layers:** The pooling layers (e.g., max-pooling) reduce the spatial dimensions of the feature maps obtained from convolution. Pooling layers help retain essential information while reducing computational complexity.
**Activation Functions:** The activation functions (e.g., ReLU) introduce non-linearity to the model. They help the network learn complex relationships within the data.
**Fully Connected Layers:** The fully connected layers process the flattened feature maps from earlier layers to make predictions. These layers combine information and produce the final output, such as class probabilities.
**Training:** The training process involves forward and backward passes. During training, the network adjusts its internal parameters (weights and biases) to minimize the loss function, making predictions more accurate.
**Feature Visualization:** CNN activates different filters to detect features in an image.
**Hierarchical Feature Extraction:** CNNs learn to extract hierarchical features, starting from simple edges and shapes and progressing to more complex and abstract features.
**The Output:** The final output of the CNN corresponds to the predicted class or category and the result is obtained through the network's computations.

The neural network goes through these steps, finally generating a predictive algorithm.

An assessment 114 is then performed using the validation dataset 111 to determine whether the training of the CNN model 112 has been successful. If the validation step fails, then the model may be fed additional training data 110a and undergo a further assessment round 114a with a new validation dataset 111a, this can continue until the CNN model successfully reaches the threshold for the validation step. A custom callback can be included to save the best model based on validation accuracy. Custom callbacks represent known tools which may be used in training a CNN or any deep learning model. They allow for customising and monitoring the training process by defining specific actions at various stages during training.

If the untrained CNN model 112 successfully meets the threshold for validation, it can be classified as a trained CNN model 115 which is able to diagnose potential diseases and conditions that can be detected from the breath-print data acquired from EBC. For example, such diseases and conditions capable of diagnosis include, but are not excluded to, lung cancer, pneumonia, asthma, interstitial lung disease and chronic obstructive pulmonary disease. The output is configured and presented on a GUI, preferably on a mobile application. An alternative, is the output is stored on a cloud-based server such as Amazon Web Services (AWS), Microsoft Azure, Google Cloud Platform (GCP), IBM Cloud, Heroku or Firebase, for later use or further analysis. Cloud-based servers can offer a robust means for building, deploying, and maintaining a flexible system for providing clinicians with real-time predictions for patients for example with lung cancer, pneumonia or other health conditions based on exhaled breath data. In order for the method and system S100 to be employed on a mobile application an Internet of Things (IoT) chip or module maybe required. Such examples include, but are not excluded to, ESP8266 and ESP32, Raspberry Pi, Arduino MKR Series, Particle, Nordic Semiconductor nRF Series.

The single point data values 107 are also capable of training a statistical Al model, such as a Linear Discriminant Analysis (LDA) model 118. LDA models are used to reduce dimensionality and improve classification accuracy by finding the optimal linear combination of features that best separates different classes in a dataset. Similarly, as seen with the untrained CNN artificial intelligence model 112, the single point data values 107 are split into a training dataset 116 and a validation dataset 117. The training dataset 116 is then used to train the model, and an alternative assessment 119 is performed using the validation dataset 117 to determine whether the training of the untrained LDA model 118 has been successful. If the validation step fails, then the model may be fed additional training data 116a and undergo a further assessment round 119a with a new validation dataset 117a. A custom callback can be included to save the best model based on validation accuracy. The cycle may be repeated as many times as required until the untrained LDA model 118 successfully passes a threshold of success with the validation dataset 117. Once the untrained LDA model 118 has successfully met the threshold for validation, the trained LDA model 120 can positively diagnose diseases and conditions from breath-prints collected from the EBC sample.

Optionally, the LDA model and CNN model may be combined via model ensembling to generate a fused model 121 with an even greater prediction power for identification of a diseased individual and diagnosing of conditions.

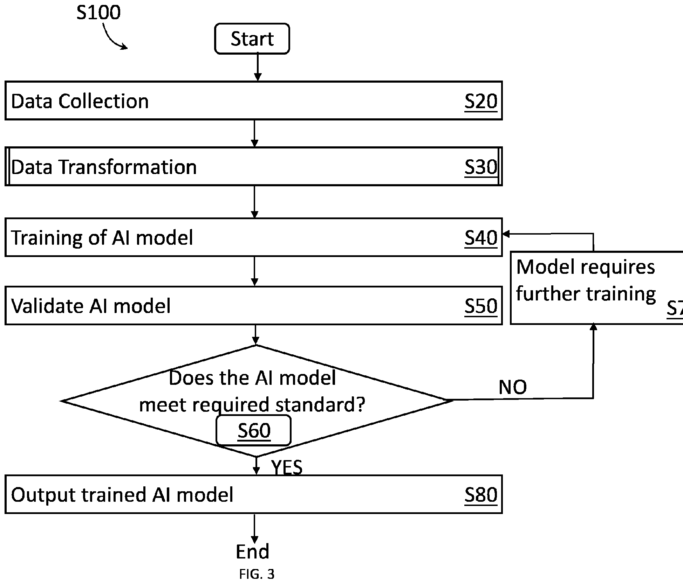

FIG. 3 identifies a method S100 of training an Al model for the diagnostics of respiratory diseases using breath print profiles from EBC. The Al model can be validated and tested with the ability to train the model further with additional iterations of training and validation data steps, until the Al model satisfies the ability to correctly interpret breath prints.
- In step S20, the data is collected from a sample of healthy individuals and those with known health conditions. Preferably, the data collected is from EBC but alternative collection samples may be used instead or in combination with. Samples may include, but are not excluded to sputum and/or blood and/or pleural fluid and/or saliva and/or nasal swab and/or throat swab samples.
- In step S30, the data collected is transformed into a format suitable for the Al model to process.
- In step S40, the Al model will be trained on a subset of the data collected in S20. The Al model is trained to recognise patterns in the data that is associated with healthy individuals and individuals with known health conditions.
- In step S50, the Al model is validated to ensure that it is able to distinguish between healthy individuals and those with health conditions, as well as identifying what the health condition is. To validate the Al model, an alternative subset of collected data, which was unseen during the training step S40, is used. The Al model receives the data only, with no information relating to the health of the individual, and must predict the diagnosis for each individual.
- In step S60, the Al model is assessed on how well it predicted the unseen data in the validation step. If the Al model does not meet an acceptable level of prediction on the validation data, then the process proceeds to step S70. If the Al model does meet an acceptable level of prediction on the validation data, the process proceeds to step S80.
- In step S70, the Al model is required to be trained with more data. The method returns to step S40, with new data used to train the Al model further. This loop can continue until the performance of the Al model meets an acceptable level at step S60.
- In step S80, the trained Al model is the output. The Al model can now be used to quickly and efficiently to diagnose individuals. Applications for such an Al model can be used to diagnose respiratory conditions, such as lung cancer, pneumonia, asthma, interstitial lung disease, chronic obstructive pulmonary disease etc. from an individual's breath-print.

The method described in FIG. 3 can be used to train deep learning Al model such as a Convolutional Neural Network (CNN) models, statistical Al models, such as a Linear Discriminant Analysis (LDA) and combinations of deep learning and statistical Al models.

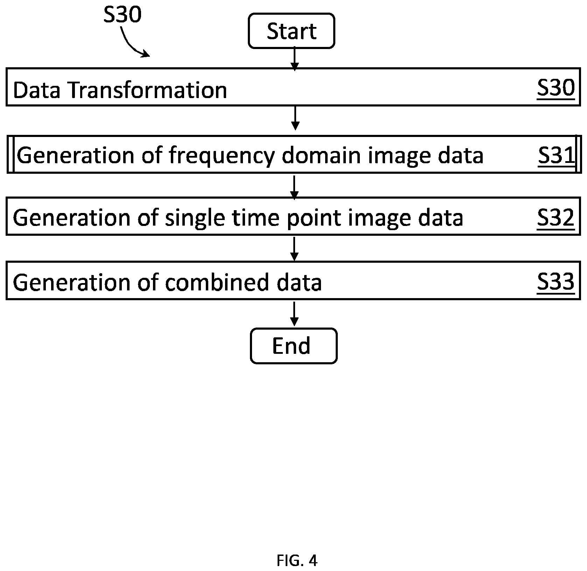

FIG. 4 illustrates a more detailed method S30 of transformation of the data input required to train a deep learning Al model for the diagnosis of respiratory diseases using breath print profiles from EBC.
- In step S31, any form of time domain data collected during step S20 is converted into frequency domain data. For example, CO₂ contents over a two-minute period can be transformed by Fast Fourier Transforms into frequency domain graph.
- In step S32, single point data values are converted to images, such as a QR codes or fractal images.
- In step S33, the single point data value images are combined with the corresponding frequency domain data images. This combined data is split into a training and validation dataset to be used in steps S40 and S50.

It can be appreciated that steps S31, generation of frequency domain data, and S32, generation of single point image data, can occur in parallel or one after the other with either being the first step. The combining data step S33 occurs after steps S31 and S32 have been completed.

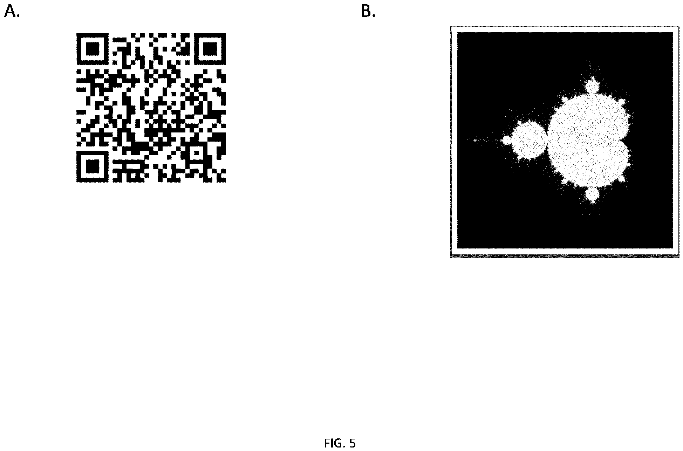

FIG. 5A shows an example of a generated QR code from a single point data value which occurs at step S32. The contents of a QR code can always be "visualised" using a camera-phone, which will show the contents of the QR code. When the QR code is read by a camera, it displays the embedded single point data values which can be used in the CNN model. FIG. 5B shows an example of a generated Mandelbrot fractal image from a single point data value. The fractal image allows for the single point data value to be encrypted by the formula and can only be read if you have the appropriate decryption key. For the images generated in FIG. 5A and 5B, the single point data value 107 is extracted from the tabular data 103 generated by an Inflammacheck^{®} device, but it could also be relevant data points e.g., metadata related to the patient or additional test results and measurements.

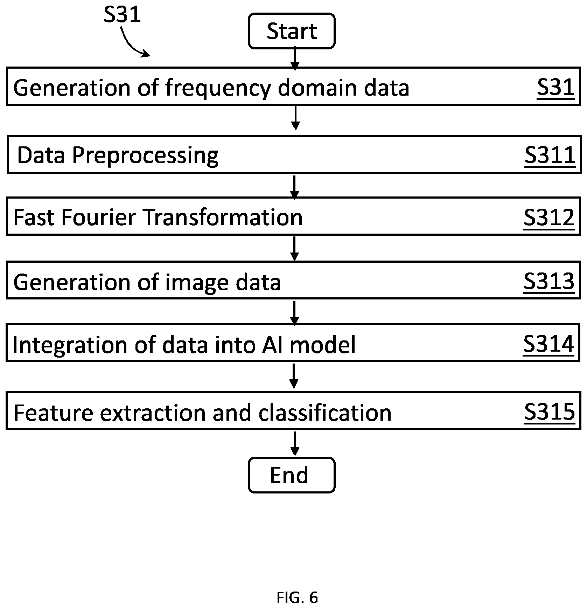

FIG. 6 illustrates a more detailed method S31 for generating the frequency domain data required to train a deep learning Al model.
- In step S311, the time series data, for example concentration of CO₂, or breath pressure, or breath flow rate over a given time period, is converted into a format suitable for FFT. This formatting task may include handling missing values and/or normalizing data and/or organising the layout for FFT. Domain-specific knowledge about medical data interpretation and clinical relevance may also be used to format the data in an appropriate way.
- In step S312, FFT method is carried out on the time domain data in order to analyse frequency components. The FFT parameters, such as window size and overlap, may be tailored depending on the context.
- In step S313, the FFT results is converted into frequency domain graph, effectively representing the frequency information as visual data.
- In step S314, the generated frequency domain graphs are then integrated into the architecture of the deep learning Al model for further analysis.
- In step S315, the frequency domain graphs serve as features for the AI model which can subsequently perform classification based on these features.

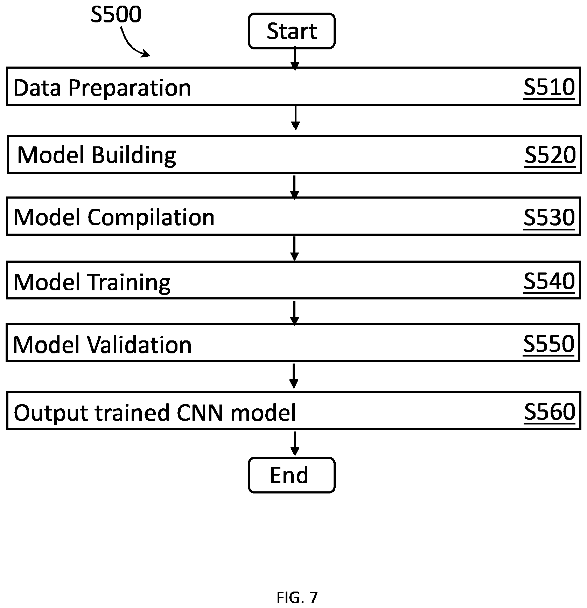

FIG. 7 illustrates a method S500 of building and training a deep learning Convolution Neural Network Al (CNN) model for the diagnostics of respiratory diseases using breath print profiles from EBC.
- In step S510, the input data collected at S20 is prepared for and image data is loaded into the Al Model. The images are resized to a specific width and height and the training data is augmented using advanced data augmentation techniques, including rotation, shifting, shearing, zooming, and flipping. The Synthetic Minority Over-sampling Technique (SMOTE) is used to address any class imbalance in the datasets.
- In step S520, the CNN model is constructed using high-level neural networks Application Programming Interface (API), such as the Sequential API. The model comprises convolutional layers which increase in complexity, followed by max-pooling layers to extract features from the images. Global max-pooling reduces spatial dimensions and extracts the most important features. Fully connected layers further process the features. The model ends with an output layer having at least four units, corresponding to at least four classes in the dataset, using a softmax activation function.
- In step S530, the CNN model is compiled using the Stochastic Gradient Descent (SGD) optimizer and categorical cross-entropy loss. The model is configured to monitor validation accuracy and save the best model (i.e. the highest diagnostics accuracy) during training. The selection and fine-tuning of optimisation algorithms can have a significant impact on training outcomes.
- In step S540, the CNN model is trained on the augmented and SMOTE training data generated in S510. During the training, the model monitors validation accuracy and saves the best model to enable custom callbacks to identify the best model. The CNN model runs for as many epochs as necessary, until it has passed through the entire training-set data.
- In step S550, the CNN model undergoes the validation step to ensure that it is able to distinguish between healthy and unhealthy, as well as identifying such health conditions in the unhealthy subset. The CNN model is validated using unseen data from S510. The model computes test accuracy, indicating how well the model generalizes to new, unseen data.
- In step S560, the trained CNN model is the output. The CNN model can now be used to quickly and efficiently to diagnose individuals.

This CNN model essentially creates a deep learning model that is able to classify images into distinct categories. It is able to handle data preprocessing, class imbalance, and applies data augmentation techniques to improve model generalisation. The model's architecture involves convolutional layers for feature extraction and fully connected layers for classification. Training is monitored to ensure that the model doesn't overfit and to capture the best-performing model. Finally, the model's accuracy on unseen test data is evaluated to assess its effectiveness in classifying images into the desired categories.

Converting breath waveform data into Fourier transforms and feeding them into CNN networks can have several advantages over feeding different non-aligned waveforms directly into CNN networks:
1. **Alignment of Features:** Fourier transforms provide a consistent representation of breath waveforms by extracting frequency-domain features. This alignment can make it easier for the CNN to learn relevant patterns and relationships, as it deals with a standardized input format.
2. **Dimension Reduction:** Fourier transforms often result in a lower-dimensional representation of the data compared to raw waveforms. This can reduce the computational complexity of the CNN and prevent overfitting, especially when there is limited data.
3. **Feature Extraction:** The Fourier transform highlights specific frequency components, which can be informative for breath analysis. This feature extraction can improve the CNN's ability to detect relevant patterns related to respiratory conditions or anomalies.
4. **Noise Reduction:** The frequency-domain representation can filter out noise or unwanted variations in the waveform data, potentially leading to more robust and accurate analysis results.
5. **Efficient Processing:** CNNs are well-suited for processing grid-like data, and the Fourier transforms can be organized into a structured input format, making the CNN's architecture more compatible with the data.
6. **Interpretability:** The frequency-domain representation might provide interpretable features related to the underlying physiology or characteristics of the breath waveforms.
7. **Transfer Learning:** Pre-trained CNN models, which have shown success in various image-related tasks, can potentially be fine-tuned more effectively when using Fourier-transformed data, as they are accustomed to working with structured inputs.

However, it's essential to consider potential disadvantages as well:
1. **Information Loss:** The Fourier transform may discard some fine-grained temporal information present in the raw waveforms, which could be relevant in certain applications.
2. **Complexity:** Implementing the Fourier transform and ensuring that the resulting data is compatible with CNN input can add some complexity to the preprocessing pipeline.

In summary, converting breath waveform data into Fourier transforms and feeding them into CNN networks can be advantageous for certain applications due to feature alignment, dimension reduction, and noise reduction. However, the choice depends on the specific goals of the analysis and the trade-offs between feature representation and interpretability.

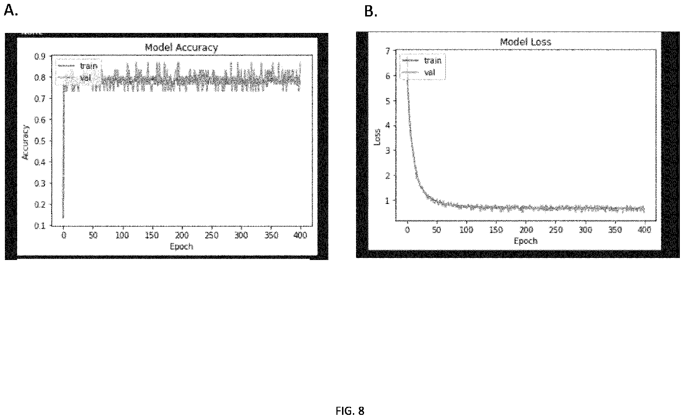

FIG. 8A and 8B are a model accuracy graph and model loss graph respectively from a CNN model. The model accuracy graph in FIG. 8A displays the accuracy of the CNN model on the training and validation datasets over the course of epochs. The accuracy represents the percentage of correctly classified samples in relation to the total number of samples. The graphs depicted here show the performance metrics of the CNN model trained on three classes, although it can be appreciated that any number of classes may be used. The model loss graph in FIG. 8B illustrates the error of a model on the training and validation datasets over many epochs. The loss represents a measure of how far off the model's predictions were from the actual target values. The cross-validation accuracy of the model shown here was greater than 86.67%.

FIGs. 8A and 8B illustrate that the model achieves a balance between effectively learning from the training data and generalizing to new data, resulting in a well-fitted model for the given task. The aim is for both the training and validation loss to be as low as possible, which means our CNN model is learning well and can recognize things effectively.

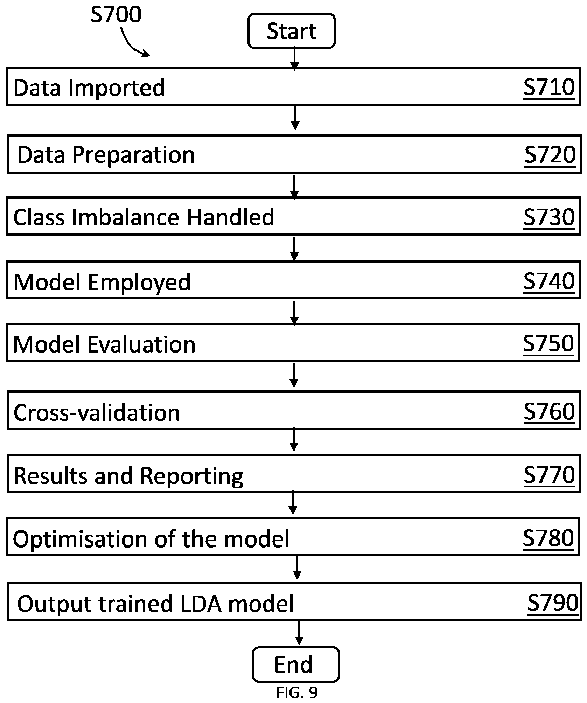

FIG. 9 illustrates a method S700 of building and training a statistical Linear Discriminant Analysis (LDA) machine learning model for the diagnostics of respiratory diseases using breath print profiles from EBC.
- In step S710, the initial dataset is imported, along with any necessary required. This dataset likely contains medical data related to lung health.
- In step S720, the data is prepared for analysis to form a variable (X) and a target variable (y). Standardisation is applied to the feature data to ensure that all features have the same scale in order to comply with the machine learning algorithms.
- In step S730, issues with class imbalance within the dataset, e.g. when one class, such as a specific disease, has significantly fewer examples than others. The step involves the use of Synthetic Minority Over-sampling Technique (SMOTE) to oversample the minority class and balance the class distribution.
- In step S740, the Machine Learning model is deployed, specifically a Linear Discriminant Analysis (LDA) classifier, to make predictions using the sample dataset. LDA is used after applying SMOTE to handle the balanced data.
- In step S750, the model is evaluated. Various evaluation metrics are used to assess the model's performance. These metrics include, but are not restricted to, accuracy, confusion matrix, sensitivity (recall), specificity, false positive rate, precision, negative predictive value, and the Matthews correlation coefficient. These metrics provide insights into how well the model can classify data into different groups.
- In step S760, the model is cross-validated, preferably using a stratified k-fold approach, to ensure that the model's performance is robust and not heavily influenced by the way in which the data is split.
- In step S770, the code reports various metrics and values related to the model's performance, including true positives, true negatives, false positives, and false negatives. These values are essential for understanding how well the model is performing and where it may need improvement.
- In step S780, a hyperparameter tuning technique, such as a grid search, is performed in order to find the best hyperparameters for the LDA model. Such techniques involve exploring a variety of combinations of solver and shrinkage values in order to optimise the model's performance.
- In step S790, the trained LDA model is the output. The LDA model can now be used to quickly and efficiently to classify individuals into different disease classification groups.

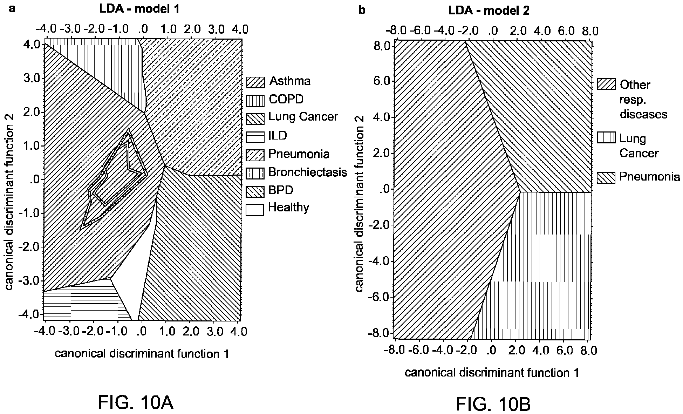

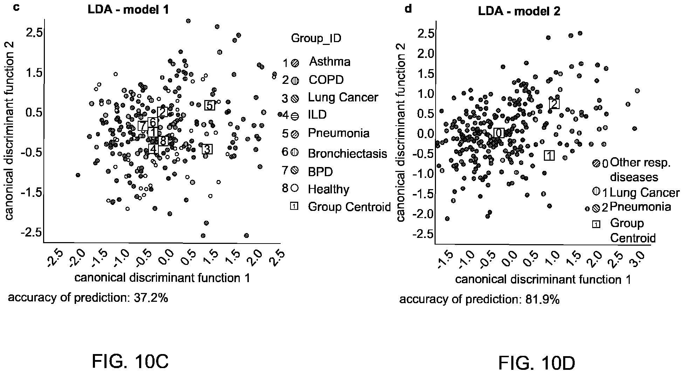

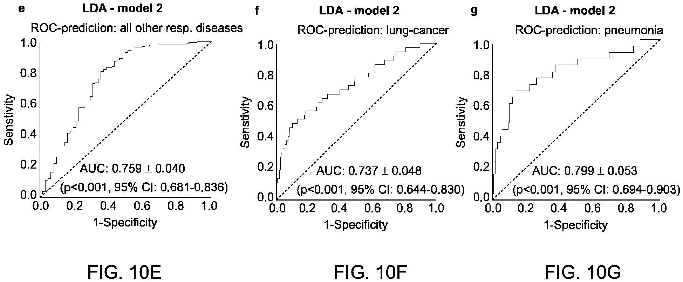

FIG. 10A-G illustrates the diagnostic prediction effectiveness of two different LDA machine learning models, LDA-model 1 and LDA-model 2. The LDA-model 1 elucidates how Inflammacheck^{®} variables discriminate among seven respiratory conditions and healthy individuals. LDA-model 2 explores the discriminative capacity of Inflammacheck variables when comparing "other respiratory conditions" (including asthma, Chronic Obstructive Pulmonary Disease (COPD), Interstitial Lung Disease (ILD), Bronchiectasis, and Breathing Pattern Disorder (BPD) grouped together) with variables specific to lung cancer and those from pneumonia patients. FIG. 10A and 10B show territorial maps illustrating the approximate data spread for LDA-model 1 and LDA-model 2, respectively. The territorial maps offer a visual representation of the data's structure and the effectiveness of each LDA model in discriminating between the input data. FIG. 10C and 10D, the distribution of individual data points, segmented by disease condition, is shown for each LDA model. FIG. 10C, displays the results of a multivariate analysis of LDA-model 1, where-by the centroids for asthma (group 1), COPD (group 2), ILD (group 4), Bronchiectasis (group 6), and BPD (group 7) are closely clustered together. In contrast, participants with pneumonia (group 5) and lung cancer (group 3) exhibit greater separation from these centroids. The proximity of centroids in LDA-model 1 reflects a poorer discriminatory ability, as each lung condition and healthy individuals retain individual classifications. This model accurately predicts 30.9% of the cross-validation dataset and 37.2% of individual lung conditions in the hold-out test set for LDA-model 1. FIG. 10D combines groups 1, 2, 4, 6, and 7 together in LDA-model 2. When data groups corresponding to asthma, COPD, ILD, Bronchiectasis, and BPD are combined to form group 0, the LDA-model 2 effectively distinguishes group 0 from group 1 (pneumonia) and group 2 (lung cancer). The cross-validation accuracy for correctly identifying these three groups from each other is 79.1%, with an accuracy exceeding 81.9% recorded for the hold-out dataset. This dataset was kept separate during the model training stages. FIG. 10E-G illustrates the ROC analysis which measures the performance of LDA-model 2 for classifying the dataset into either group 0 (all other respiratory disease), group 2 (lung cancer) and group 1 (pneumonia). LDA-model 2 successfully achieved an Area Under the Curve (AUC) of 0.737 for identifying lung cancer individuals, FIG. 10F, and for identifying individuals with pneumonia, FIG. 10G.

The disclosed examples can be realised by a computer of a system or apparatus. These examples can be implemented by any device configured to execute instructions, or any dedicated hardware that is capable of carrying out all or a portion of the functionality. Disclosure is provided of hardware (e.g., a processor such as a central processing unit (CPU) or a microprocessor unit (MPU)) configured to read out and executes a program recorded on a memory device to perform the functions of the disclosed examples. For this purpose, the program is provided to the computer for example via a network or from a recording medium of various types serving as the memory device (e.g., a computer-readable medium such as a non-transitory computer-readable medium). The steps of the disclosed methods may be performed in any suitable order, or simultaneously where possible.

Although the disclosed subject matter has been described using specific terminology relating to apparatus features and/or method features, it is to be understood that the claimed subject matter is not necessarily limited to the examples disclosed. The scope of the following claims is to be accorded the broadest interpretation so as to encompass all such modifications and equivalent structures and functions. The advantages disclosed may relate to several of the examples that are disclosed.

### Example

In an example study, an observational cross-sectional investigation utilizing the Inflammacheck^{®} device, included participants with respiratory conditions and healthy volunteers. A scatter-plot matrix of the variables tested can be seen in FIG. 1. Recruitment occurred at a single large NHS university hospital, encompassing inpatient and outpatient settings. Adult participants (≥16 years) with confirmed diagnoses of respiratory diseases such as asthma, COPD, bronchiectasis, ILD, primary lung cancer, pneumonia, and breathing pattern disorders were included. Healthy volunteers, also ≥16 years, without respiratory or significant medical conditions were part of the study. All participants needed to be in a stable condition, able to provide informed consent, and unlikely to experience harm from testing. Exclusions applied to individuals with co-existing conditions preventing spirometry completion (for asthma, COPD, and healthy controls), those who hadn't undergone spirometry in the past 12 months, pneumonia patients requiring supplemental oxygen, and those unable to perform study procedures.

## Claims

1. A method (S100) of obtaining training data for an existing artificial intelligence, Al, model to process data for diagnostics purposes, the method comprising the steps of:
providing (S20) an input dataset comprising of a first set of data including data representative of a sampling device and a second set of data including time domain data representative of a patient sample; and
processing (S30) the input dataset to obtain a transformed dataset to provide as training dataset to the existing Al model.

2. The method (S100), according to Claim 1, wherein the step of processing (S30) the input dataset comprises:
generating (S31) frequency domain data from time domain data in the second dataset;
generating single point image data (S32) from the first dataset; and
combining (S33) the frequency domain dataset with the generated single point image data.

3. The method (S100) according to claim 1 or claim 2, further comprising the steps of:
providing (S40) to the existing Al model a first subset of the transformed dataset; and
validating (S50) the Al model using a second subset of the transformed dataset, to obtain a trained Al model for diagnostics.

4. The method (S100) according to any one of the preceding claims, wherein the sampling device is adapted to combine measurements made on exhaled breath in its gas phase with measurement of H₂O₂ levels made on exhaled breath condensate in its liquid phase.

5. The method (S100) according to any one of claims 2 to 4, wherein generating (S31) frequency domain data comprises:
preprocessing of the time domain data (S311) to obtain preprocessed data suitable for a Fourier Transformation;
applying a Fourier Transformation (S312) to the preprocessed data to obtain transformed data; and
generating a frequency domain image (S313) from the transformed data.

6. The method (S100) according to claim 5, further comprising the steps of:
providing the frequency domain image to an existing Al model (S314); and
performing, by the existing Al model using the frequency domain data, feature extraction and classification (S315).

7. The method (S100) according to any one of the preceding claims, wherein the first dataset comprises a QR code image.

8. The method according to any one of the preceding claims, wherein the first data set comprises a fractal image.

9. The method (S100) according to any one of the preceding claims, wherein the diagnostics are diagnostics of a respiratory disease or condition.

10. The method (S100) according to any one of the preceding claims, wherein the existing Al model is a deep learning artificial intelligence model.

11. The method (S100) according to any one of the preceding claims, wherein the existing Al model is a statistical artificial intelligence model.

12. The method (S100) according to any one of the preceding claims wherein the existing artificial intelligence model employs advanced data augmentation techniques.

13. A program which, when executed by a computer, causes the computer to perform a method according to any preceding claim.

14. A computer-readable storage medium storing a program according to claim 13.

15. A system comprising a processor configured to perform the steps of:
providing (S20) an input dataset comprising of a first set of data including data representative of a sampling device and a second set of data including time domain data representative of a patient sample; and
processing (S30) the input dataset to obtain a transformed dataset suitable for providing as training data to an existing artificial intelligence, Al, model.
